# EUROPEAN PATENT APPLICATION

(11) **EP 3 786 967 A1**
(43) Date of publication of application: **03.03.2021**
(21) Application number: 20190172.5
(22) Date of filing: 10.08.2020
(51) Int. Cl.: G16H 20/17, G16H 40/63, G16H 40/67, A61M 5/168, A61M 5/14, A61M 5/172

(54) **COUNTER DEVICE**

(30) Priority: 27.08.2019 FI 20195706
(71) Applicant: Monidor OY, 90590 Oulu (FI)
(72) Inventor: Savola, Mikko, 90590 Oulu (FI); Puurunen, Veli-Matti, 90590 Oulu (FI); Puolitaival, Seppo, 90590 Oulu (FI)
(74) Representative: Kolster Oy Ab

(57) **Abstract**

Apparatuses and methods are disclosed. The invention relates to a method in a drip counter device comprising: obtaining (400) information on the drip counter device; controlling (402) the transceiver to obtain information about the infusion liquid dosing plan of the person the infusion liquid is to be administered; controlling (404) the transceiver to transmit the obtained information to a system monitoring device; receiving (406) device parameters to administer infusion liquid according to the dosing plan; monitoring (408) the administering of the infusion liquid; comparing (410) the administering with the dosing plan; controlling (412) the transceiver to transmit information on monitoring or comparison results to the system monitoring device and controlling (414) the user interface to display comparison results.

## Description

### Background

Counter devices or drip counters are used in medicine in connection with intravenous infusion therapy. By means of a drip counter, it is made sure that a patient receives the desired amount of infusion liquid within the desired time.

Dripping rate of infusion liquid refers to the rate at which infusion liquid is administered for the patient. Conventionally, the dripping rate is indicated as the measured number of drips per minute, but the dripping rate may also be indicated as millilitres per hour, if the volume of one drip is known in advance and if the aforementioned minute-specific number of drips has been determined.

Conventionally the dripping rate of counter devices or drip counters is set by hand by the operator of the counter, such as a nurse, using a mechanical clamp, for example. A drip counter does conventionally only monitoring i.e. it counts how many drops/minute is administered to a patient. Operator can set limits/alarms so that when the dropping rate is under/higher than the limit an alarm may be given. Conventionally there is a separate clamp in a pipe, and the clamp is used for controlling a flow in the pipe. A clamp and a monitor are not typically related to each other in any way.

### Brief description

According to an aspect of the present invention, there are provided apparatuses of claims 1 and 8.

According to an aspect of the present invention, there are provided methods of claims 12 and 16.

One or more examples of implementations are set forth in more detail in the accompanying drawings and the description below. Other features will be apparent from the description and drawings, and from the claims. The embodiments and or examples and features, if any, described in this specification that do not fall under the scope of the independent claims are to be interpreted as examples useful for understanding various embodiments of the invention.

### Brief description of the drawings

In the following the invention will be described in greater detail by means of preferred embodiments with reference to the accompanying drawings, in which
Figure 1 illustrates an example of a use of a drip counter device;
Figure 2 illustrates an example of a drip counter device;
Figure 3 illustrates an example of an environment where a drip counter device applying some embodiments of the invention may be used;
Figure 4 is a flowchart illustrating an embodiment;
Figure 5 illustrates an example of the operation of a drip counter device;
Figure 6A and 6B are flowcharts illustrating some embodiments of the invention; and
Figure 7 illustrates an example of an apparatus employing some embodiments of the invention.

### Detailed description of some embodiments

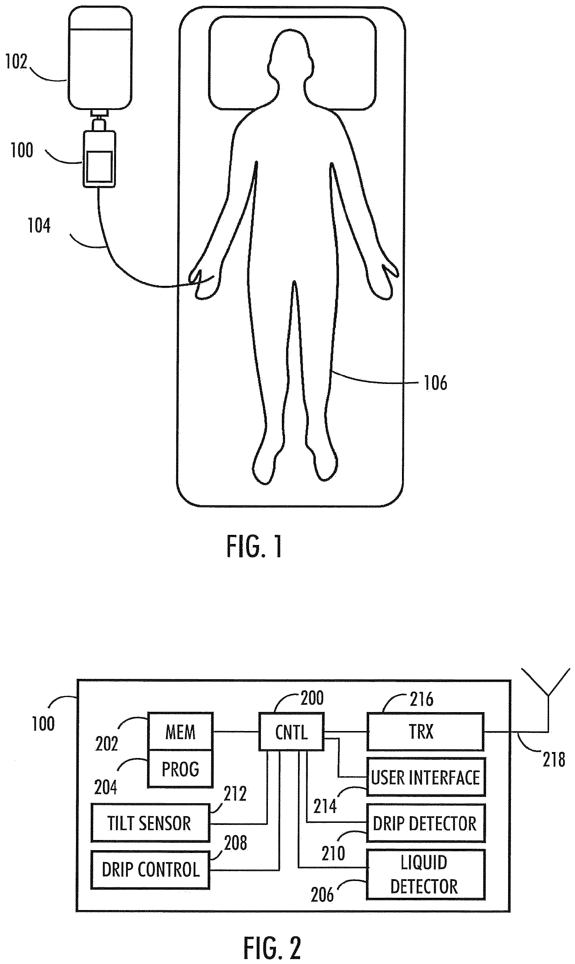

Fig. 1 illustrates an example of a use of a drip counter device. Fig. 1 shows a drip counter 100, to which a container 102 of infusion liquid is attached. The drop counter is configured to administer 104 infusion liquid to a patient 106 so that the patient acquires a desired amount of infusion liquid within the desired time. The drip counter typically controls the dripping rate of infusion liquid. The dripping rate refers to the rate at which infusion liquid is administered for the patient.

A drip counter typically comprises a drip detector which is configured to measure the dripping rate. The drip detector may be realized, for example with an optical detector, such as an LED (light emitting diode) light source and a phototransistor as its pair. Modern drip counter devices are such that the device may show on a display the number of the drips in a given time, or the dripping rate such as drops/minute or drops/hour, dropped volume counted or the drops counted. In addition, a drip counter may feature adjustable alarm thresholds by means of which the device provides an alarm for the nursing staff if the dripping rate of the infusion liquid is not at the desired level or if a preset maximum amount of infusion liquid has been administered, that is, dripped.

Current drip counter devices are very simple drop counters. A typical device is not connected to hospital database or linked to the web. An advanced drop counter may store use information to an external memory as history information. It is known that some devices are nowadays connected to the external database or cloud to send measured or monitored data. There are also some infusion pumps which are connected to the database for updating device information.

The user of the present devices meets many practical challenges and problems to set a device to start monitoring. First the medical information of the infusion liquid is needed to be available in the device. The device manufacturer can provide data and load it to the device. However, the devices are not on-line connected and when new infusion liquid types and forms are taken into use, the user may need to feed data manually. Second, the patient's medicine list must be input to the device. The data could be available in the hospitals patient's database but because the device is not connected, the list must be input manually from a paper or from a computer. Third issue relates to follow-up of a patient's planned care plan (infusion liquid) and monitored infusion given. Due to lack of connection to the databases the comparison may be challenging due to the fact that the plan may be changed during the care. For example, a user may modify and reset infusion dropping speed and time to reach daily targets. Fourth, an issue relates to comparing the realized infusion liquid administering to the planned care plan designed by a medical doctor.

Fig. 2 illustrates an embodiment. The figure illustrates a simplified example of a drip counter device 100 applying embodiments of the invention.

It should be understood that the apparatus is depicted herein as an example illustrating some embodiments. It is apparent to a person skilled in the art that the apparatus may also comprise other functions and/or structures and not all described functions and structures are required. Although the apparatus has been depicted as one entity, different modules and memory may be implemented in one or more physical or logical entities.

The device 100 of the example includes a control circuitry 200 configured to control at least part of the operation of the apparatus. The control circuitry may be realized with at least one processor, for example.

The device may comprise a memory 202 for storing data. Furthermore, the memory may store software 204 executable by the control circuitry or processor 200. The memory may be integrated in the control circuitry.

In an embodiment, the device may comprise a detector 206 configured to determine presence of infusion liquid to be administered. The detector may be a mechanical or optical switch connected to the control circuitry or processor 200, for example.

In an embodiment, the device may comprise a drip control unit 208, which controls the amount of liquid to be administered for the patient. The device further comprises a drip detector 210 configured to measure the dripping rate. The device may further comprise a tilt sensor 212 which may determine if the device is not in upright position, in which case the operation of the device may be incorrect.

The device may comprise a transceiver 216 which may be connected to an antenna 218.

The various above described apparatuses of the device 100 may all be connected to and operate under the control of the control circuitry or processor 200. For example, the speaker and display of the user interface may indicate alarms under the control of the control circuitry or processor.

The transceiver 216 may be a wireless transceiver utilizing known radio technology. An example of suitable radio technology is Bluetooth®. Example of other possible technologies are wireless local area network (WLAN or WiFi), worldwide interoperability for microwave access (WiMAX), ZigBee® and various cellular communication technologies well known in the art.

In an embodiment, the transceiver is a wired transceiver connected with a wired connection to a suitable network such as the Internet.

The device may comprise a user interface 214, which may comprise a display, a keypad and a speaker, for example. The display may be touch sensitive in which case a kay pad or keyboard is not necessarily required. In an embodiment, the user interface is at least partly realized in a remote monitoring apparatus connected to the device via the transceiver in a wired or wireless manner.

In an embodiment, the control circuitry or processor 200 may control the transceiver to make a connection to a patient database of the hospital the drip control device is located in. Contact parameters (such as contact address, authorization passwords, etc.) may have been fed into the control circuitry or processor beforehand by operating personnel.

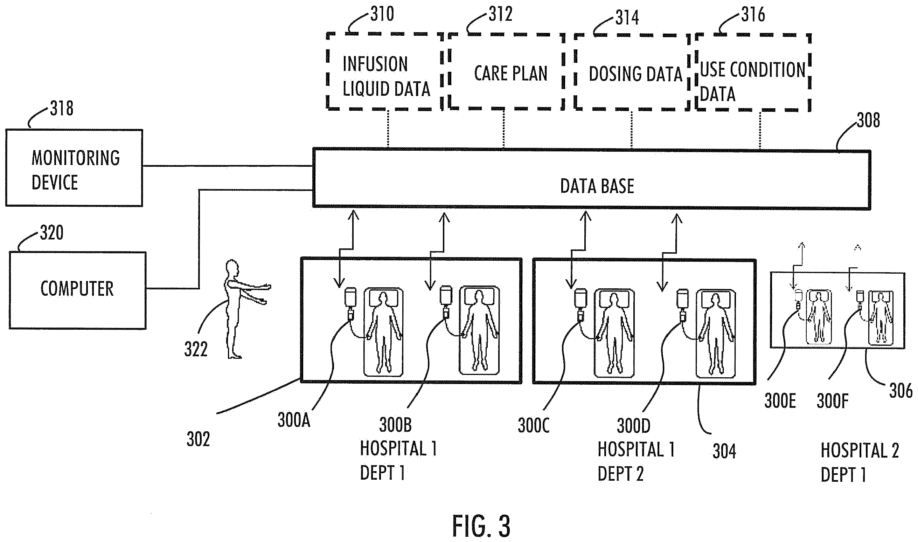

Fig. 3 illustrates an embodiment. The figure illustrates an environment where a drip counter device applying embodiments of the invention may be used.

In the example of Fig. 3, there are drip counters 300A, 300B installed and in use in a hospital 1 department 1 302, drip counters 300C, 300D installed and in use in a hospital 1 department 2 304, and drip counters 300E, 300F installed and in use in another hospital 2 department 1 306. In each department there may be one or more operators 322 of drip counters. The operators may be nurses, for example.

Utilizing the transceivers of the drip counters, the drip counters may communicate with a data base 308. The data base may comprise various information, such as infusion liquid data 310 (data of different liquids used in the hospitals), care plans 312 and medical dosing data 314 of patients of the hospitals, and use condition data of drip counters. The use condition may comprise data related to the environment of drip counters such as the department of a hospital, the room number, bed number where the drip counter is used, for example.

In the example of Fig. 3, there is a monitoring device 318 connected to the data base 308 and configured to communicate with the drip counter devices 300A - 300F. The monitoring device may be a computer or a corresponding data processing unit such as a tablet or a smart phone. There may further be another computer 320 connected to the data base. The computer may be used by medical personnel such as medical doctors to monitor the operation of various equipment.

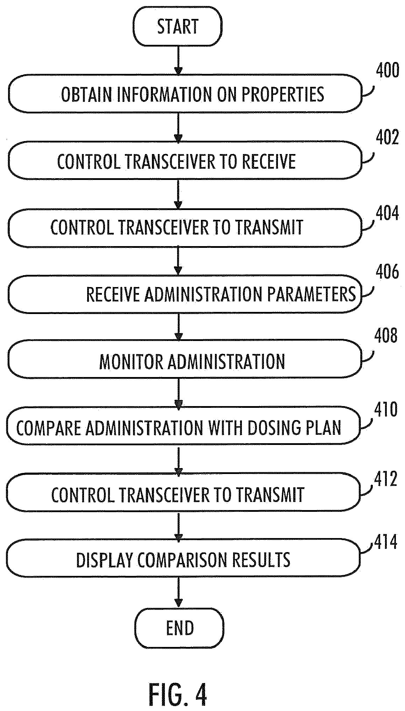

Fig. 4 is a flowchart illustrating some embodiments. Fig. 4 illustrates the operation of a drip counter device 100.

In step 400 of Fig. 4, the drip counter device is configured to obtain information on usage properties of the drip counter device. The usage properties may comprise accessories connected to the drip counter device. The accessories may be pipes and clamps attached to the device. The usage properties may comprise information on the site where the device is located, such as the department of a hospital, the room number, bed number and such. At the same time date and time information may be obtained. Some of this information may be given by the operator of the device using the user interface. As mentioned, the user interface may at least partly be located in a remote apparatus connected to the drip counter device in a wired or wireless manner. Some information may be read by the control circuitry or processor from memory or using sensors. In an embodiment, the drip counter device is also configured to receive information on the person or operator operating the drip counter device. This information may be given by the operator of the device using the user interface. In an embodiment, the device may recognize the operator using a sensor, for example.

In step 402 of Fig. 4, the drip counter device is configured to control the transceiver to obtain information on infusion liquid dosing plan of the person the infusion liquid is to be administered. The control circuitry or processor of the device may utilize the transceiver to contact database 308 of the hospital. The database may store care plans, medicine lists and dosing plans of patients of the hospital. The operator of the drip counter device may key in the identification of the patient so that corresponding data may be retrieved from the database.

In an embodiment, the drip counter device may retrieve patient's medicine list and care plan from the database.

In step 404 of Fig. 4, the drip counter device is configured to control the transceiver to transmit information on received and obtained information to the monitoring device 318. The monitoring device may be a computer or a corresponding data processing unit such as a tablet or a smart phone. The information may comprise data on the infusion liquid data, patient's care plan and medicine list. In addition, the information may comprise data related to the use situation of the device, such as the department of a hospital, the room number, bed number and such.

In step 406 of Fig. 4, the drip counter device is configured to receive device parameters to administer infusion liquid according to the dosing plan. The operator of the device may set the parameters. In an embodiment, the drip counter device is configured to show on a display the parameters.

In an embodiment, the drip counter device may be configured to show the parameters to administer infusion liquid according to the dosing plan or a changed plan, so that the operator of the device can set the infusion arrangement according the parameters. For example, if the dosing plan indicates that the patient connected to the drip counter is to receive 500 ml of infusion liquid in 5 hours, the drip counter device may calculate or receive from a monitoring device that the feed rate of 100 ml/hour or XX drops in minute is to be applied. The device may show the feed rate as a parameter on its display so that the operator can control a mechanical clamp roller so that the feeding speed is correct.

In step 408 of Fig. 4, the drip counter device is configured to monitor the administering of the infusion liquid. The control circuitry or processor 200 may utilize the drip detector 210 to determine how the infusion liquid is administered.

In step 410 of Fig. 4, the drip counter device is configured to compare the administering with the dosing plan. The control circuitry or processor 200 may calculate the administered amount of liquid based on data retrieved from the drip detector and compare the result to amount defined in the dosing plan.

In step 412, the drip counter device is configured to control the transceiver to transmit information to the monitoring device 318. The information may comprise monitored data (such as the amount of infusion liquid administered in given time frame) or results of the comparison and possibly other data.

In step 414 of Fig. 4, the drip counter device is configured to control the user interface of the drip counter device to display comparison results. The display of the user interface may show how the realized administering of the infusion liquid compares to the dosing plan. As mentioned, the user interface may at least partly be located in a remote apparatus connected to the drip counter device in a wired or wireless manner.

The proposed solution improves the quality of monitoring how the dosing plan is followed.

In an embodiment, before the above described procedure, the drip counter device may be configured to determine the presence of infusion liquid to be administered. The control circuitry or processor 200 may control the detector 206 to determine presence of infusion liquid.

In an embodiment, the drip counter device is configured to determine based on the monitoring of the administering of the infusion liquid that the dosing plan will not be fulfilled. The control circuitry or processor 200 may calculate that with the given drip control values the required amount of infusion liquid cannot be delivered in required time. Based on the determination, the drip counter device may be configured to generate an alarm, which may be shown utilizing the user interface. The alarm may be visible, audible or both. In an embodiment, the alarm is displayed in a remote apparatus connected to the drip counter device in a wired or wireless manner.

In an embodiment, the drip counter device is configured to determine, based on the monitoring, updated parameters for the drip control 208. The update parameters may be indicated utilizing the user interface in connection with the alarm.

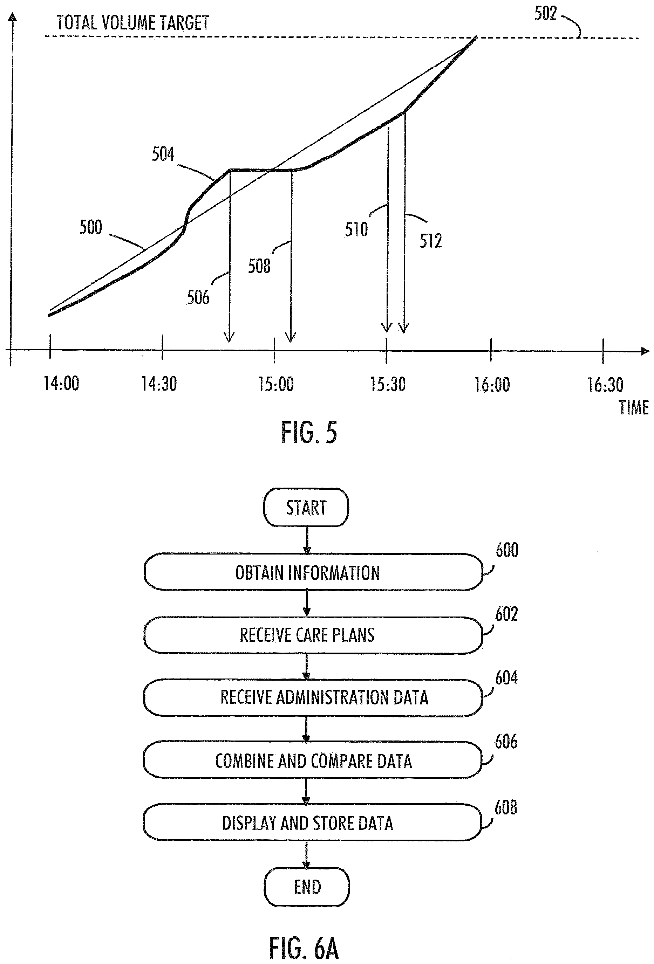

Fig. 5 illustrates above example. The figure shown dosing plan 500 of a patient for time frame 14:00 - 16:00, where time is on x-axis and liquid volume on y-axis. The total liquid volume target to be delivered in the given time frame is indicated with 502.

When the administration is initiated at 14:00, the monitored administering of infusion liquid 504 follows the plan quite well, until at 14:50, 506, the infusion administration has stopped for some reason until 15:05, 508. At 15:30, 510, the drip counter device monitoring the administration indicates an alarm that the dosing plan will likely not be fulfilled. At 15:35, 512, a new dosing setting is set to the drip control (proposed by the drip counter device in connection with the alarm and set by the operator of the device). With the new setting the target volume is achieved in the desired time frame.

In an embodiment, the drip counter device may generate an alarm of a given type. If the device has not received an acknowledgement of the alarm using the user interface within a given time limit, it may be configured to change the alarm type, for example to a louder sound or a different visual appearance. This relates to a problem where an alarm first goes unnoticed. If infusion is interrupted for too a long time a cannula may be is stuffed and there is need to perform re-cannulation at the infusion site. Clinicians may have problems in re-cannulation for patients with bad veins. Infusion may stop and continue normally soon and not causing problem, but in case infusion is stopped too long time, re-cannulation is needed. To this purpose changing alert type is useful to increase the noticeability of possible problems.

In an embodiment, the drip counter devices alert immediately with a low priority alert (such as an alert symbol of yellow traffic light) when infusion is stopped. The device may indicate the time that infusion has been stopped. The device may give a medium priority alarm (such as an audio alarm and traffic light blinking yellow) if the time lapsed from the alarm is longer than T1 when the risk that cannula is stuffed is likely. The device may give a high priority alarm (such as more frequently audio alarms) if the time lapsed from the alarm is longer than T2 and the risk that cannula is stuffed is very likely.

T1 and T2 may be set by clinicians and could be for example in range of 5 to 15 minutes for T1 and in range of 15 to 30 minutes for T2, for example.

In an embodiment, the drip counter device is configured to generate an alarm with of a given type, wherein the type of the alarm is selected based on the received information on the person the infusion liquid is administered.

As an example, the drip counter device detects that the infusion is stopped and it will make an alarm. The stopped infusion may be due to the technical or infusion liquid handling problem (infusion bag is empty, a pipe is flattened, monitoring is not working). One other reason may be that the cannula set to a vein is stuffed. The risk of stuffing of the cannula may depend on the patient and cannula setting. The drip counter device may take that into consideration when making an alarm. For example, older people may have thin and hard veins so that cannula stuffing is more likely than in younger and healthier people. It also means that setting of a cannula (again) is challenging so it is important to try to avoid that if possible and the system alarm prioritization can help in this.

An operator of the drip counter device such as a care person or a clinician can set a person to be monitored to be "higher risk" for cannula stuffing. If so, set the system will prioritize the alarm. The alarm may be a medium priority alarm at once or the time frame when the alarm type is increased may be shorter time than in "normal cases", for example 20-50% shorter.

The prioritization can be also made automatically based on persons age, weight, infusion type, infusion liquid used etc. This data will be available from the database 308 or input separately.

In an embodiment, the drip counter device may determine that an alarm is to be given. It may determine that a previous alarm had been given within a given time frame and select the type of the alarm based on the determination.

In an embodiment, the alarm type and/or frequency of changing alarm type may depend on determined information obtained by comparing the administration with the dosing plan. For example, if the drip counter device compares dosing plan and monitored data and gives an alert with low priority alert (such as an alert symbol of yellow traffic light, for example) if there is XX % variation from care plan. A medium priority alarm (such as audio alarm and traffic light blinking yellow, for example) may be given if there is YY % variation from Care plan. Here XX and YY may be set by operator or a clinician and could be for example in range of 15-25% for XX and in range of 25-50% for YY.

In an embodiment, monitoring system or device may also notice that infusion cannot be executed according to dosing plan safely. For example, if 2 litres should be infused in 2 hours due to slow infusion in the beginning of infusion the monitoring system or device may give a warning - it may not be safe to infuse such amount of liquid in so short time.

Fig. 6A is a flowchart illustrating some embodiments of the invention. Fig. 6A illustrates the operation of a monitoring device 318. In general, the monitoring device (or the computer 320) is configured to monitor one or more drip counter devices. The monitoring device maybe a processing device or computer comprising a transceiver and user interface.

In step 600 of Fig. 6A, the monitoring device is configured to control the transceiver of the device to obtain information on person or persons operating one or more drip counter devices and persons infusion liquid is to be administered utilizing the one or more drip counter devices and properties of the one or more drip counter devices and environment in which they are used. The transceiver may utilize wired or wireless communication methods. The information may be obtained from the data base 308 or from the drip counter devices.

In step 602 of Fig. 6A, the monitoring device is configured to control the transceiver of the device to obtain information on care plan and infusion liquid dosing plan of one or more persons infusion liquids are to be administered. In an embodiment, the information may be obtained from the data base 308.

In step 604 of Fig. 6A, the monitoring device is configured to control the transceiver to receive information on monitored administering of the infusion liquid received. In an embodiment, the information is sent from the one or more more drip counter devices.

In step 606 of Fig. 6A, the monitoring device is configured to combine and compare the monitored administering with the dosing plan.

In step 608 of Fig. 6A, the monitoring device is configured to control the user interface to display information related to the monitored administering and the dosing plans of the one or more drip counter devices. In an embodiment, the monitoring device is configured to show status of all connected drip counter devices on the one overview screen providing a quick and reliable way of monitoring the devices. The information may also be stored in the database 308.

On hospital wards there may be several patients receiving intravenous infusion at the same time. Especially in night-time there may be a low number of personnel to check infusions. When the any of the personnel does not have time to check infusions by going to the patient, problems may occur. Infusion may run too fast or it can stop without any alarm. These problems can cause complications. The proposed solution eases the management of drip counters.

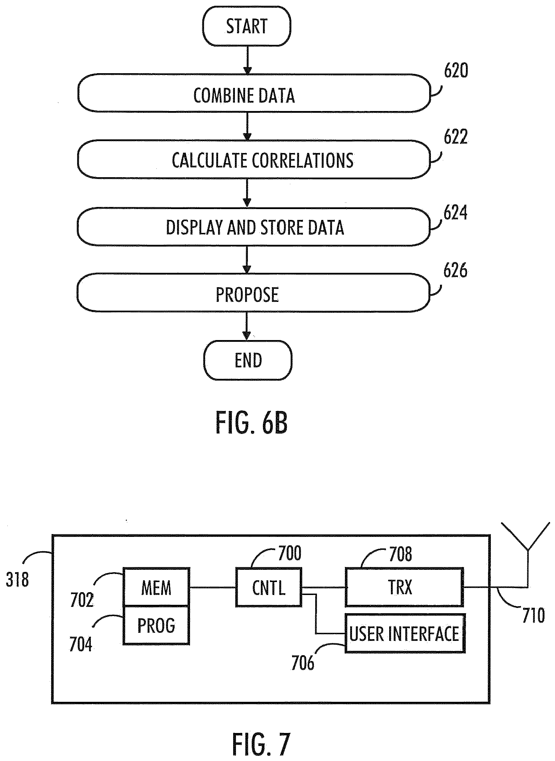

Fig. 6B is another flowchart illustrating some embodiments of the invention. Fig. 6B illustrates another example of the operation of the monitoring device 318.

In step 620 of Fig. 6B, the monitoring device is configured to combine the monitored administering with information on persons infusion liquid is administered and properties of the one or more drip counter devices and environment in which they are used to obtain statistics data related to individual persons infusion liquid is administered and to multiple persons infusion liquid is administered, to persons operating the drip counter devices and to properties of the one or more drip counter devices and to environment in which they are used.

In step 622 of Fig. 6B, the monitoring device is configured to calculate correlations between differences in the care plans and infusion liquid dosing plans and monitored administering.

In step 624 of Fig. 6B, the monitoring device is configured to control the user interface to display results. The information may also be stored in the database 308.

In an embodiment, the monitoring device may provide proposals for actions to decrease differences between in the care plans and infusion liquid dosing plans and monitored administering.

For example, the actions may comprise one or more of the following: checking drip counter device functionality, checking drip counter device accessories, checking the medicine or liquid packages, checking care practices, checking user instruction, checking the use settings and practices, proposing in dedicated plan instructions, proposing instruction related to use conditions.

The proposed solution enables analyzing how the realized care plans (infusion liquid) are related to planned care plans in the different use cases and circumstances. It is valuable to know if there are correlation to device types, setting, users, departments, medicine, liquid type and manufacturer, pipe and clamp types and manufacturers to improve reliability and accuracy and possible to guide users to check the device differently or inform and alarm the user differently or guide a care plan designer (such as a medical doctor) to do a plan differently.

Fig. 7 illustrates an embodiment. The figure illustrates a simplified example of a monitoring device or apparatus 318 applying embodiments of the invention.

It should be understood that the apparatus is depicted herein as an example illustrating some embodiments. It is apparent to a person skilled in the art that the apparatus may also comprise other functions and/or structures and not all described functions and structures are required. Although the apparatus has been depicted as one entity, different modules and memory may be implemented in one or more physical or logical entities.

The apparatus 318 of the example includes a control circuitry or processor 700 configured to control at least part of the operation of the apparatus.

The apparatus may comprise a memory 702 for storing data. Furthermore, the memory may store software 704 executable by the control circuitry or processor 700. The memory may be integrated in the control circuitry.

The apparatus may comprise a transceiver 708 configured to communicate in a wired or wireless manner with other devices. The transceiver may be connected to an antenna arrangement 710. The transceiver may be a radio transceiver utilizing Bluetooth®, the universal mobile telecommunications system (UMTS) radio access network (UTRAN or E-UTRAN), long term evolution (LTE, the same as E-UTRA), wireless local area network (WLAN or WiFi), worldwide interoperability for microwave access (WiMAX), or any combination thereof. The interface may be an interface to a wired communication system. The transceiver may be operationally connected to the control circuitry 700.

The software 704 may comprise a computer program comprising program code means adapted to cause the control circuitry or processor 700 of the apparatus to perform the embodiments described above and in the claims.

The apparatus may comprise a user interface 706. the user interface may comprise a display, a keyboard or keypad, for example. The display may also be touch sensitive.

The steps and related functions described in the above and attached figures are in no absolute chronological order, and some of the steps may be performed simultaneously or in an order differing from the given one. Other functions can also be executed between the steps or within the steps. Some of the steps can also be left out or replaced with a corresponding step.

The apparatuses or controllers able to perform the above-described steps may be implemented as an electronic digital computer, which may comprise a working memory (RAM), a central processing unit (CPU), and a system clock. The CPU may comprise a set of registers, an arithmetic logic unit, and a controller. The controller is controlled by a sequence of program instructions transferred to the CPU from the RAM. The controller may contain a number of microinstructions for basic operations. The implementation of microinstructions may vary depending on the CPU design. The program instructions may be coded by a programming language, which may be a high-level programming language, such as C, Java, etc., or a low-level programming language, such as a machine language, or an assembler. The electronic digital computer may also have an operating system, which may provide system services to a computer program written with the program instructions.

As used in this application, the term 'circuitry' refers to all of the following: (a) hardware-only circuit implementations, such as implementations in only analog and/or digital circuitry, and (b) combinations of circuits and software (and/or firmware), such as (as applicable): (i) a combination of processor(s) or (ii) portions of processor(s)/software including digital signal processor(s), software, and memory(ies) that work together to cause an apparatus to perform various functions, and (c) circuits, such as a microprocessor(s) or a portion of a microprocessor(s), that require software or firmware for operation, even if the software or firmware is not physically present.

This definition of 'circuitry' applies to all uses of this term in this application. As a further example, as used in this application, the term 'circuitry' would also cover an implementation of merely a processor (or multiple processors) or a portion of a processor and its (or their) accompanying software and/or firmware. The term 'circuitry' would also cover, for example and if applicable to the particular element, a baseband integrated circuit or applications processor integrated circuit for a mobile phone or a similar integrated circuit in a server, a cellular network device, or another network device.

The computer program may be in source code form, object code form, or in some intermediate form, and it may be stored in some sort of carrier, which may be any entity or device capable of carrying the program. Such carriers include a record medium, computer memory, read-only memory, and a software distribution package, for example. Depending on the processing power needed, the computer program may be executed in a single electronic digital computer or it may be distributed amongst a number of computers.

The apparatus may also be implemented as one or more integrated circuits, such as application-specific integrated circuits ASIC. Other hardware embodiments are also feasible, such as a circuit built of separate logic components. A hybrid of these different implementations is also feasible. When selecting the method of implementation, a person skilled in the art will consider the requirements set for the size and power consumption of the apparatus, the necessary processing capacity, production costs, and production volumes, for example.

In an embodiment, the apparatus applying some embodiments of the invention comprises means for obtaining information on the drip counter device, means for obtaining information on infusion liquid dosing plan of the person the infusion liquid is to be administered, means for transmitting information on obtained information to a system monitoring device, means for receiving device parameters to administer infusion liquid according to the dosing plan, means for monitoring the administering of the infusion liquid, means for comparing the administering with the dosing plan means for control transmission of information on monitoring or comparison results to the system monitoring device, means for controlling displaying comparison results.

In an embodiment, the apparatus applying some embodiments of the invention comprises means for obtaining information on person or persons operating one or more drip counter devices and persons infusion liquid is to be administered utilizing the one or more drip counter devices and properties of the one or more drip counter devices and environment in which they are used, means for obtaining information on care plan and infusion liquid dosing plan of one or more persons infusion liquids are to be administered, means for receiving information on monitored administering of the infusion liquid received, means for combining and comparing the monitored administering with the dosing plan, and means for displaying information related to the monitored administering and the dosing plans of the one or more drip counter devices.

It will be obvious to a person skilled in the art that, as the technology advances, the inventive concept can be implemented in various ways. The invention and its embodiments are not limited to the examples described above but may vary within the scope of the claims.

## Claims

1. A drip counter device for counting infusion liquid drips, the device comprising
a drip detector;
a user interface;
a transceiver;
at least one processor;
at least one memory including computer program code;
the at least one memory and the computer program code configured to, with the at least one processor, cause the device at least to perform:
obtain information on the drip counter device;
control the transceiver to obtain information on infusion liquid dosing plan of the person the infusion liquid is to be administered, wherein the dosing plan comprises the amount of infusion liquid to be administered in a given time interval;
control the transceiver to transmit information on obtained
information to a system monitoring device;
receive device parameters to administer infusion liquid according to the dosing plan;
monitor the administering of the infusion liquid;
compare the administering with the dosing plan;
determine based on the monitoring of the administering of the infusion liquid that the dosing plan will not be fulfilled;
control the transceiver to transmit information on monitoring or comparison results to the system monitoring device;
control the user interface to display comparison results.

2. The device of claim 1, the at least one memory and the computer program code configured to, with the at least one processor, cause the apparatus further to perform:
generate an alarm based on the determination.

3. The device of claim 2, the at least one memory and the computer program code configured to, with the at least one processor, cause the apparatus further to perform:
determine, based on the monitoring, updated device parameters;
indicate the updated parameters.

4. The device of claim 2, the at least one memory and the computer program code configured to, with the at least one processor, cause the apparatus further to perform:
indicate the time lapsed from the alarm.

5. The device of claim 2, the at least one memory and the computer program code configured to, with the at least one processor, cause the apparatus further to perform:
generate an alarm with of a given type;
change the alarm type if an acknowledgement of the alarm has not been detected by the user interface within a given time limit.

6. The device of claim 2, the at least one memory and the computer program code configured to, with the at least one processor, cause the apparatus further to perform:
receive information on the person the infusion liquid is to be administered;
generate an alarm with of a given type, wherein the type of the alarm is selected based on the received information on the person the infusion liquid is administered.

7. The device of claim 2, the at least one memory and the computer program code configured to, with the at least one processor, cause the apparatus further to perform:
determine that a previous alarm had been given within a given time frame;
select the type of the alarm based on the determination.

8. An apparatus for monitoring one or more drip counter devices, the apparatus comprising
a user interface;
a transceiver;
at least one processor;
at least one memory including computer program code;
the at least one memory and the computer program code configured to, with the at least one processor, cause the device at least to perform:
control the transceiver to obtain information on person or persons operating one or more drip counter devices and persons infusion liquid is to be administered utilizing the one or more drip counter devices and properties of the one or more drip counter devices and environment in which they are used;
control the transceiver to obtain information on care plan and infusion liquid dosing plan of one or more persons infusion liquids are to be administered, wherein the dosing plan comprises the amount of infusion liquid to be administered in a given time interval;
control the transceiver to receive information on monitored administering of the infusion liquid received, the information indicating that the dosing plan will not be fulfilled;
combine and compare the monitored administering with the dosing plan;
control the user interface to display information related to the monitored administering and the dosing plans of the one or more drip counter devices.

9. The apparatus claim 8, the at least one memory and the computer program code configured to, with the at least one processor, cause the apparatus further to perform:
combine the monitored administering with information on persons infusion liquid is administered and properties of the one or more drip counter devices and environment in which they are used to obtain statistics data related to individual persons infusion liquid is administered and to multiple persons infusion liquid is administered, to persons operating the drip counter devices and to properties of the one or more drip counter devices and to environment in which they are used;
calculate correlations between differences in the care plans and infusion liquid dosing plans and monitored administering;
control the user interface to display results.

10. The apparatus of claim 9, the at least one memory and the computer program code configured to, with the at least one processor, cause the apparatus further to perform:
provide actions proposals to decrease differences between in the care plans and infusion liquid dosing plans and monitored administering.

11. The apparatus of claim 9, wherein the actions comprise one or more of the following:
checking drip counter device functionality, checking drip counter device accessories, checking the medicine or liquid packages, checking care practices, checking user instruction, checking the use settings and practices, proposing in dedicated plan instructions, proposing instruction related to use conditions.

12. A method in a drip counter device comprising:
obtaining information on the drip counter device;
control the transceiver to obtain information on infusion liquid dosing plan of the person the infusion liquid is to be administered, the dosing plan comprising the amount of infusion liquid to be administered in a given time interval;
controlling the transceiver to transmit obtained information to a system monitoring device;
receiving device parameters to administer infusion liquid according to the dosing plan;
monitoring the administering of the infusion liquid;
comparing the administering with the dosing plan;
determining based on the monitoring of the administering of the infusion liquid that the dosing plan will not be fulfilled;
controlling the transceiver to transmit information on monitoring or comparison results to the system monitoring device;
controlling the user interface to display comparison results.

13. The method of claim 12, further comprising:
generating an alarm based on the determination.

14. The method of claim 13, further comprising:
determining, based on the monitoring, updated device parameters;
indicating the updated parameters.

15. The method of claim 13, further comprising:
generating an alarm with of a given type;
changing the alarm type if an acknowledgement of the alarm has not been detected by the user interface within a given time limit.

16. A method for monitoring one or more drip counter devices, the method comprising:
controlling the transceiver to obtain information on person or persons operating one or more drip counter devices and persons infusion liquid is to be administered utilizing the one or more drip counter devices and properties of the one or more drip counter devices and environment in which they are used;
controlling the transceiver to obtain information on care plan and infusion liquid dosing plan of one or more persons infusion liquids are to be administered, wherein the dosing plan comprises the amount of infusion liquid to be administered in a given time interval;
controlling the transceiver to receive information on monitored administering of the infusion liquid received, the information indicating that the dosing plan will not be fulfilled;
combining and comparing the monitored administering with the dosing plan;
controlling the user interface to display information related to the monitored administering and the dosing plans of the one or more drip counter devices.

17. The method of claim 16, further comprising:
combining the monitored administering with information on persons infusion liquid is administered and properties of the one or more drip counter devices and environment in which they are used to obtain statistics data related to individual persons infusion liquid is administered and to multiple persons infusion liquid is administered, to persons operating the drip counter devices and to properties of the one or more drip counter devices and to environment in which they are used;
calculating correlations between differences in the care plans and infusion liquid dosing plans and monitored administering;
controlling the user interface to display results.

18. A computer program comprising instructions for causing an apparatus to perform any of the method steps of claims 12 to 17.
